Europäisches Patentamt

European Patent Office

Office européen des brevets

(11) Numéro de publication : **0 383 675 B1**

(12)

# FASCICULE DE BREVET EUROPEEN

(45) Date de publication du fascicule du brevet :
25.08.93 Bulletin 93/34

(51) Int. Cl.⁵ : **C07C 19/00, C07C 17/38**

(21) Numéro de dépôt : **90400391.0**

(22) Date de dépôt : **13.02.90**

(54) **Procédé de séchage d'hydrocarbures, son application à la préparation des chloromethanes.**

(30) Priorité : **16.02.89 FR 8902024**

(43) Date de publication de la demande :
**22.08.90 Bulletin 90/34**

(45) Mention de la délivrance du brevet :
**25.08.93 Bulletin 93/34**

(84) Etats contractants désignés :
**AT BE CH DE DK ES FR GB GR IT LI LU NL SE**

(56) Documents cités :
**US-A- 4 307 260
KIRK-OTHMER: "Concise Encyclopedia of
Chemical Technology", 1985, pages 374-375,
John Wiley & Sons, New York, US**

(73) Titulaire : **ELF ATOCHEM S.A.
4 & 8, Cours Michelet La Défense 10
F-92800 Puteaux (FR)**

(72) Inventeur : **Masini, Jean-Jacques
35, Rue Jean Penet
F-69630 Chaponost (FR)**
Inventeur : **Ghenassia, Elie
20, Avenue Albert 1er de Belgique
F-38100 Grenoble (FR)**
Inventeur : **Commandeur, Raymond
Le Rocher - Avenue de Vénaria
F-38220 Vizille (FR)**
Inventeur : **Clair, René
La Vrignoise, Saint-Julien
F-13500 Martigues (FR)**
Inventeur : **Guillaumenq, Jean-Louis
16, Résidence des Termes Hauts de
Saint-Jean
F-13110 Port De Bouc (FR)**

EP 0 383 675 B1

## Description

La présente invention concerne un procédé de séchage d'hydrocarbures et son application à la production des chlorométhanes.

L'invention concerne plus particulièrement les hydrocarbures éventuellement halogénés qui contiennent de l'eau et de l'acide chlorhydrique. On a déjà décrit le séchage du perchloréthylène par une solution de chlorure de calcium (C. Abstracts, Vol. 99-177 849 d) et en l'absence d'acide chlorhydrique. On a aussi décrit le séchage du chloroforme $CHCl_3$ et du tétrachlorure de carbone $CCl_4$ après leur purification par extraction à l'eau (C. Abstracts, Vol. 62-2227 e) avec du chlorure de calcium ($CaCl_2$) mais toujours en l'absence d'acide chlorhydrique. Le problème qui intéresse la demanderesse est de sécher les hydrocarbures contenant de l'acide chlorhydrique et de l'eau sans enlever l'acide chlorhydrique. On trouve ce problème dans la synthèse des chlorométhanes en sortie du réacteur de chloration où les chlorméthanes sont mélangés à de l'acide chlorhydrique (chaque fois qu'on substitue un hydrogène par un chlore on sous-produit une mole d'acide chlorhydrique) et à l'eau qui est introduite dans le procédé comme impureté des matières premières. Il est nécessaire d'enlever l'eau pour éviter son accumulation dans le procédé et aussi pour éviter l'obturation des tuyaux par la glace et les hydrates. L'acide chlorhydrique est récupéré puis valorisé par conversion en chlore dans une réaction de DEACON ou une réaction d'oxychloration. Si on enlève l'eau de ce mélange de chlorométhanes par condensation on risque, vu la grande solubilité de l'acide chlorhydrique dans l'eau, d'obtenir une solution d'acide chlorhydrique dans l'eau difficile à séparer ensuite. L'inconvénient risque d'être le même si on veut séparer l'eau de ce mélange par un agent desséchant.

Le KIRK-OTHMER, concise encyclopedia of chemical technology, 1985 page 374-375, John Wiley New-York, décrit en page 374 des agents séchants pour des liquides et pouvant être de l'oxyde de baryum, du chlorure de calcium, du sulfate de calcium, du chlorure de lithium des perchlorates du $P_2O_5$ et des hydroxydes de potassium ou de sodium. Mais sans précision quant aux liquides ni au séchage sélectif.

Le brevet US 4307 260 décrit le séchage d'un mélange de $CH_3Cl$, diméthyléther, HCl et eau par passage dans une solution d'acide chlorhydrique à - 18° C. Ce procédé retient l'eau et HCl. A sa grande surprise, la demanderesse a trouvé qu'on pouvait sécher sélectivement les hydrocarbures contenant de l'acide chlorhydrique et de l'eau en retenant seulement l'eau.

L'invention est donc un procédé de séchage sélectif d'un mélange contenant au moins un hydrocarbure, de l'acide chlorhydrique et de l'eau, caractérisé en ce qu'on le met en contact avec un desséchant choisi parmi (i) les sulfates, les chlorures ou les perchlorates métalliques anhydres ou (ii) le pentoxyde de phosphore jusqu'à ce que le desséchant ait retenu seulement la majeure partie de l'eau.

Bien que l'invention concerne tout hydrocarbure, on l'applique avantageusement au benzène et ses dérivés alkylés ou polyalkylés, c'est-à-dire le benzène substitué par une ou plusieurs chaînes hydrocarbonées, linéaires ou ramifiés ayant chacune jusqu'à 8 atomes de carbone. On peut citer par exemple le benzène, le toluène, le xylène, l'isopropylbenzène, le styrène et l'éthylbenzène. L'invention concerne aussi des hydrocarbures halogénés.

L'hydrocarbure halogéné peut contenir du fluor, du chlore ou du brome ou deux ou trois de ces éléments, être saturé ou insaturé, c'est-à-dire comporter une ou plusieurs doubles liaisons ou une ou plusieurs triples liaisons, ou toute combinaison de ces possibilités. De préférence l'hydrocarbure halogéné contient entre 1 et 4 atomes de carbone. L'invention est particulièrement utile pour les chlorométhanes et pour les hydrocarbures chlorés à deux atomes de carbone. Parmi les hydrocarbures chlorés à deux carbones, citons avantageusement le 1-2 dichloroéthane, le chlorure de vinyle, le 1,1,1-trichloroéthane, le 1,1,2-trichloroéthane, le trichloroéthylène et le perchloroéthylène. L'hydrocarbure halogéné peut être un mélange de plusieurs hydrocarbures halogénés, il peut aussi être dans un solvant. La quantité d'acide chlorhydrique peut être quelconque, la quantité d'eau aussi. Cependant, la quantité d'eau est avantageusement inférieure à 1 % en poids et de préférence inférieure à 0,1 %.

On ne sortirait pas du cadre de l'invention pour des quantités d'eau plus importantes mais l'invention n'aurait plus d'intérêt économique. En effet pour des quantités essentiellement de l'ordre de quelques pour cent, ou au-dessus, il est plus simple d'utiliser des séparations conventionnelles telles que distillation ou une extraction, puis d'utiliser l'invention.

Le mélange peut être gazeux ou liquide ou partiellement gazeux. La température et la pression peuvent être quelconques. On peut mettre en oeuvre l'invention pour sécher le mélange quelles que soient la température, la pression à laquelle il est disponible sans qu'il soit nécessaire de les modifier. Le mélange peut aussi contenir d'autres produits, par exemple du chlore.

Le desséchant est sous forme solide et il est séparable facilement du mélange. Il peut se présenter sous la forme de poudre ou de granulés en lit fixe ou en lit fluide. C'est un produit dont la fonction est de retenir seulement l'eau mais ni l'acide chlorhydrique, ni éventuellement le chlore s'il est présent, et bien sûr pas l'hy-

2

drocarbure halogéné. C'est un sel anhydre qui bien évidemment ne doit pas réagir chimiquement avec l'acide chlorhydrique. Les sulfates, les chlorures et les perchlorates métalliques conviennent bien. On peut utiliser, par exemple, le sulfate de calcium, le sulfate de sodium, le sulfate de cuivre, le chlorure de zinc, le chlorure de calcium, le perchlorate de barium ou le perchlorate de magnésium.

On utilise avantageusement le chlorure de calcium. La qualité du desséchant dépend de la quantité d'eau devant rester dans le mélange.

Avantageusement on utilise un chlorure de calcium dont la teneur en eau est comprise entre 0 et 25 % en poids et, de préférence, entre 0 et 12 %.

Pour obtenir un mélange ne contenant plus que quelques ppm d'eau il est nécessaire d'utiliser un desséchant essentiellement anhydre. Par exemple, pour obtenir un mélange ne contenant pas plus de 10 ppm d'eau, il est nécessaire d'utiliser un chlorure de calcium tel que sa teneur en eau soit inférieure à 5 % en poids.

Quant à la quantité de desséchant, elle dépend de la quantité totale d'eau qu'on veut retenir. Par exemple, quand on travaille en lit fixe le desséchant, qui se trouve du côté de l'entrée du flux de mélange à sécher, se sature en eau et ainsi de suite pour tout le lit. Pour assurer un bon séchage, il suffit qu'il reste suffisament de desséchant anhydre, non encore saturé en eau. Le temps de séjour du mélange à sécher avec le desséchant peut être quelconque. Avantageusement, il est inférieur à 10 minutes et de préférence compris entre 1 et 5 minutes. On ne sortirait pas du cadre de l'invention en utilisant des temps de séjour beaucoup plus longs, mais ce n'est pas nécessaire pour obtenir le résultat de l'invention.

Les temps de séjour importants correspondent à un volume important de desséchant. C'est une bonne sécurité pour obtenir un bon séchage mais cet important volume peut provoquer des pertes de charges incompatibles avec le reste du procédé. L'homme de métier peut choisir très facilement le meilleur compromis.

La présente invention concerne aussi un procédé de synthèse de chlorométhanes, caractérisé en ce qu'on met en contact, en un point quelconque du procédé, un mélange contenant au moins un chlorométhane de l'acide chlorhydrique et de l'eau avec un desséchant choisi parmi (i) les sulfates, les chlorures ou les perchlorates métalliques anhydres ou (ii) le pentoxyde de phosphore.

La synthèse des chlorométhanes consiste à préparer le chlorure de méthyle ($CH_3Cl$) puis par chloration les chlorométhanes supérieurs, chlorure de méthylène ($CH_2Cl_2$), chloroforme ($CHCl_3$) et tétrachlorure de carbone ($CCl_4$). Le $CH_3Cl$ est obtenu par chloration du méthane ou par hydrochloration du méthanol puis ce $CH_3Cl$, en tout ou partie avec éventuellement une partie des chlorométhanes supérieurs, subit une chloration par du chlore liquide ou gazeux. En sortie du réacteur de chloration on obtient un mélange de chlorométhanes d'acide chlorhydrique, d'un peu d'eau, et éventuellement d'un peu de chlore.

L'eau n'est ni un réactif ni un produit, mais elle est présente comme impureté dans les matières premières telles que le chlore et il peut en rester dans le $CH_3Cl$ venant de la réaction d'hydrochloration du méthanol.

On sépare l'HCl par distillation de ce mélange dans la colonne dite "colonne d'HCl", et on profite d'un profil de concentration favorable des traces de chlore dans cette colonne pour épuiser ces traces de chlore, par exemple, à l'aide de lampes U.V. en achevant la chloration. On obtient en pied un mélange de chlorométhanes, d'eau et encore un peu d'acide chlorhydrique mal séparé dans la colonne d'HCl. Ce mélange subit une suite de distillations pour séparer les différents chlorométhanes qui constituent la production et une partie est recyclée au réacteur de chloration pour ajuster les proportions des différents chlorométhanes.

Dans une première colonne (qui suit la colonne d'HCl), dite "colonne de chlorure de méthyle", on sort en tête le $CH_3Cl$ et la majeure partie de l'eau ainsi que les traces d'HCl et de chlore qui étaient encore présentes après la colonne d'HCl. En pied de cette colonne de $CH_3Cl$ on a un mélange des chlorométhanes supérieurs qui est à son tour séparé en ses différents composants par distillation. Bien que son point d'ébullition soit plus élevé que les chlorométhanes supérieurs, les traces d'eau sortent en tête à cause de différents azéotropes partiels et des formations d'hydrates de $CH_3Cl$ plus ou moins stables.

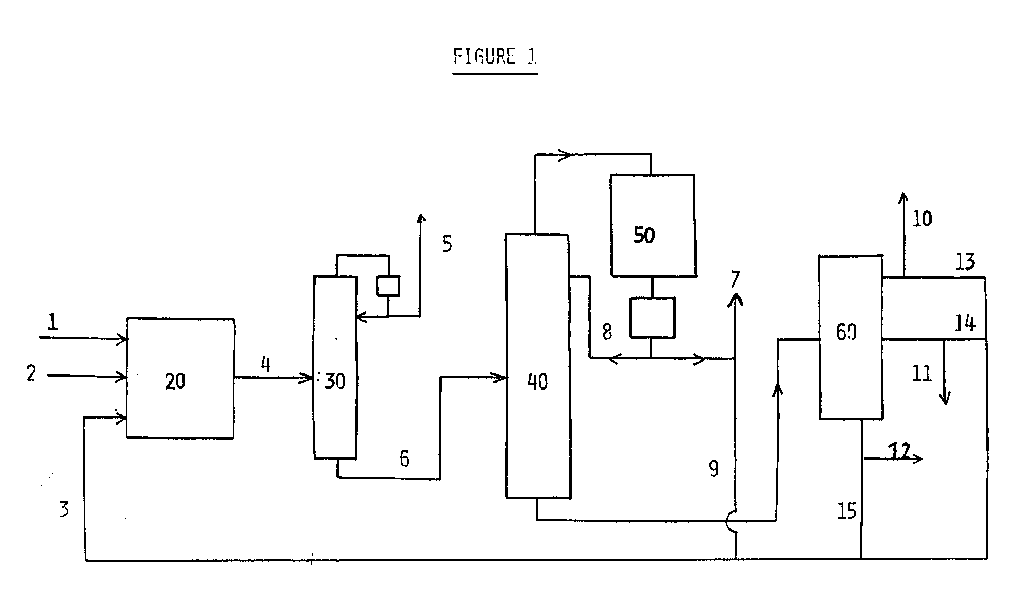

Un tel procédé de synthèse de chlorométhanes est décrit dans les brevets EP 128818, GB 2 158 067, GB 2 181 132 et GB 1 456 568 au nom de la demanderesse. On applique avantageusement l'invention au chlorure de méthyle qui vient de l'hydrochloration du méthanol ou de la chloration du méthane et au $CH_3Cl$ issu de la colonne de $CH_3Cl$ soit sur tout le flux, soit seulement sur la partie recyclée vers le réacteur de chloration. L'intérêt de l'invention est d'enlever l'eau pour éviter son accumulation dans le procédé et aussi pour éviter des problèmes de corrosion. En effet, en tête de la colonne de $CH_3Cl$, la présence simultanée d'eau, d'HCl et éventuellement de chlore, conduit à des corrosions. Selon une forme préférée de l'invention on sèche le mélange sortant de la tête de la colonne $CH_3Cl$ avant le condenseur et le pot de reflux. La figure 1 représente cette forme préférée de l'invention.

(20) est le réacteur de chloration, (30) la colonne de séparation d'HCl, (40) la colonne de $CH_3Cl$, (50) le dispositif de séchage selon l'invention et (60) le dispositif de séparation des chlorométhanes supérieurs. En (1) on introduit le $CH_3Cl$ venant de l'hydrochloration (non représentée), en (2) le chlore et en (3) les chlorométhanes recyclés. La sortie (4) est distillée en (30) et on recueille l'HCl en (5) puis le pied, contenant les chlorométhanes de l'eau et un peu d'HCl, alimente la colonne (40) par le tuyau (6). (7) représente la production de $CH_3Cl$, (8) le reflux et (9) le recyclage. En (60) on sépare les chlorométhanes supérieurs ; (10), (11) et (12) représentent respectivement la production de $CH_2Cl_2$, $CHCl_3$ et $CCl_4$. (9), (13), (14) et (15) représentent $CH_3Cl$, $CH_2Cl_2$, $CHCl_3$ et $CCl_4$ qui sont réunis dans le flux (3) et recyclés au réacteur (20).

Le desséchant est de préférence du chlorure de calcium. Le mélange à sécher sortant en tête de la colonne de $CH_3Cl$ a une température comprise entre 5°C et 60°C et de préférence entre 20 et 50°C. Sa pression est avantageusement comprise entre 1 et 12 bars absolus et de préférence entre 4 et 10 bars absolus.

La quantité d'eau peut varier dans de larges limites, elle est avantageusement inférieure à 0,5 % en poids et de préférence comprise entre 50 et 500 ppm. Quant à la quantité d'HCl, sa teneur dépend de l'efficacité de la colonne de séparation d'HCl, elle est en général inférieure à 1000 ppm et de préférence comprise entre 50 et 500 ppm.

Le mélange de tête de la colonne $CH_3Cl$ à sécher peut aussi contenir du chlore, la concentration varie selon l'efficacité du réacteur de chloration et de l'éventuelle réaction de finition qu'on peut mettre en oeuvre dans la colonne d'HCl. Cette concentration peut aller jusqu'à 10000 ppm.

La colonne de séparation du $CH_3Cl$ peut être couplée avec la colonne de séparation du $CH_2Cl_2$, ou bien on peut avoir une seule colonne sortant $CH_3Cl$ en tête et les chlorométhanes supérieurs à différents soutirages latéraux ou toute combinaison du même genre, bien connue dans la mise en oeuvre des distillations. On ne sortirait pas du cadre de l'invention en disposant le sécheur selon l'invention en tête de cette colonne sur la phase gazeuse constituée essentiellement de chlorure de méthyle.

Les exemples suivants illustrent l'invention.

EXEMPLE 1

Un sécheur est constitué d'une colonne en verre de hauteur 0,5 m et 0,45 m de diamètre qui est remplie de 30 Kg de $CaCl_2$ en grains de 3 à 8 mm sur une hauteur de 37,5 cm. La teneur en eau du $CaCl_2$ est 2,3 %. Puis on fait passer sur ce lit, de bas en haut, un flux de $CH_3Cl$ gazeux contenant de l'HCl, de l'eau et du chlore et ceci pendant 408 heures. Les résultats sont reportés sur le tableau I.

## TABLEAU I

| Pression bar absolu | Durée h | Temps de séjour | (H$_2$O) ppm | | (Cl$_2$) ppm | | (HCl) ppm | |
|---|---|---|---|---|---|---|---|---|
| | | | e | s | e | s | e | s |
| 1 | 170 | 4 mn | 70 à 105 | < 25 | 85 | 85 | 300 | 300 |
| 1 | 180 | 4 | 900 | < 30 | 500 | 490 | 300 | 290 |
| 1 | 190 | 4 | 300 | < 30 | 550 | 500 | 350 | 380 |
| 1 | 211 | 4 | 300 | < 30 | 1050 | 1050 | 350 | 350 |
| 1 | 265 | 4,5 | 310 | 25-30 | 950 | 980 | 300 | 290 |
| 1 | 297 | 3,5 | 300 | < 25 | 950 | 980 | 500 | 460 |
| 1,1 | 376 | 4 | 150 | < 25 | | | | |
| 1,5 | 383 | 4,5 | 190 | < 25 | 950 | 980 | 500 | 460 |
| 1 | 408 | 5,3 | 300 | < 25 | 2100 | 2000 | 350 | 350 |

e = entrée sécheur        s = sortie sécheur

Le temps de séjour est calculé pour le sécheur vide.

## EXEMPLE 2

On opère comme dans l'exemple 1 mais en chargeant le sécheur (en partant du bas vers le haut du lit) avec :

$$7 \text{ kg de CaCl}_2 \text{ à } 22 \quad \% \text{ d'eau}$$
$$5 \text{ kg } " \quad " \quad 12 \quad \% \quad "$$
$$8 \text{ kg } " \quad " \quad 21,5 \% \quad "$$
$$8 \text{ kg } " \quad " \quad 10 \quad \% \quad "$$
$$2 \text{ kg } " \quad " \quad 12 \quad \% \quad "$$

On opère ainsi pendant 32 heures puis on observe un perçage du lit.
Les résultats sont reportés dans le tableau II.

TABLEAU II

| Durée h | Temps de séjour mn | $(H_2O)$ ppm | | $(Cl_2)$ ppm | | (HCl) ppm | |
|---|---|---|---|---|---|---|---|
| | | e | s | e | s | e | s |
| 0 | 4,6 | 520 | 130 | 1100 | 1050 | 300 | 300 |
| 10 h 30 | 4,6 | 520 | 130 | " | " | " | " |
| 13 | 4,6 | 350 | 120 | " | " | " | " |
| 14 | 6,5 | 450 | 100 | " | " | " | " |
| 15 | 4,6 | 450 | 130 | " | " | " | " |
| 32 | 4,6 | 300 | 130 | " | " | " | " |

EXEMPLE 3

On opère comme dans l'exemple 1 mais en chargeant le sécheur (en partant du bas vers le haut du lit) avec :

$$7 \text{ kg de } CaCl_2 \text{ à } 22 \quad \% \text{ d'eau}$$
$$5 \text{ kg " } \quad " \quad 12 \quad \% \quad "$$
$$3 \text{ kg " } \quad " \quad 21,5 \% \quad "$$

On fonctionne ainsi pendant 12 heures puis on rajoute 15 kg de $CaCl_2$ à 1 % d'eau au dessus du lit. On a fonctionné au total 50 heures.

Les résultats sont reportés sur le tableau III.

EP 0 383 675 B1

TABLEAU III

| Durée h | Temps de séjour mn | $(H_2O)$ ppm | | $(Cl_2)$ ppm | | (HCl) ppm | |
|---|---|---|---|---|---|---|---|
| | | e | s | e | s | e | s |
| 0 | 4,6 | 212 | 160 | 1100 | 1050 | 300 | 300 |
| 10 | 10,5 | 255 | 160 | " | " | " | " |
| 12 | 4,6 | 270 | 150 | " | " | " | " |
| 13 | 4,9 | 230 | 25 | " | 1200 | 150 | 150 |
| 35 | 4,9 | 265 | 25 | " | 1050 | " | " |
| 40 | 4,9 | 360 | 25 | " | " | " | " |
| 50 | 4,9 | 300 | 25 | 1260 | 1300 | 130 | 140 |

EXEMPLE 4

On opère comme dans l'exemple 1 mais en chargeant le sécheur (en partant du bas vers le haut du lit) avec :

$$7 \text{ kg de } CaCl_2 \text{ à } 22 \text{ % d'eau}$$
$$5 \text{ kg } " \quad " \quad 12 \text{ % } "$$
$$4 \text{ kg } " \quad " \quad 5 \text{ % } "$$
$$14 \text{ kg } " \quad " \quad 1 \text{ % } "$$

Les résultats sont reportés sur le tableau IV.

TABLEAU IV

| Durée h | Temps de séjour mn | $(H_2O)$ ppm | | $(Cl_2)$ ppm | | (HCl) ppm | |
|---|---|---|---|---|---|---|---|
| | | e | s | e | s | e | s |
| 10 | 4,5 | 280 | 25 | 1570 | 1550 | 240 | 240 |
| 35 | 4,5 | 300 | 25 | 1550 | 1550 | 300 | 300 |

7

EXEMPLE 5 (Comparatif)

On sèche des chlorométhanes sur des tamis moléculaires. On dispose dans un tube en verre, de diamètre intérieur 13 mm et de hauteur 700 mm, 50 g de tamis moléculaire, soit 71 ml sur 540 mm de hauteur. Les chlorométhanes sont dans un flacon et s'écoulent par gravité dans la couche de tamis moléculaire à un débit variable réglé par robinet à pointeau et sont recueillis dans un erlen respirant sur un laveur à acide sulfurique concentré.

Un débit régulier de chlorométhanes est obtenu en plaçant le flacon sous une pression d'azote constante.

On utilise un tamis potassique de 3 Angstroms, sous forme de billes de 2 mm.

Les résultats sont reportés dans le tableau V dans lequel TM signifie Tamis Moléculaire.

## TABLEAU V

| Liquide testé | Débit (1/h) | Vitesse linéaire (cm/mn) | Vitesse spatiale Débit(1/h) Vol TM (1) | $H_2O$ ppm | HCl ppm |
|---|---|---|---|---|---|
| Mélange (en poids) | | | | | |
| $CH_2Cl_2$ : 17 % | 1,5 | 19 | 21 | 38 | 4 |
| $CHCl_3$ : 60 % | 2,0 | 25 | 28 | 38 | " |
| $CCl_4$ : 23 % | 2,5 | 31,5 | 35 | 33 | " |
| $H_2O$ = 85 ppm | | | | | |
| HCl = 22 ppm | | | | | |
| Mélange chloro-méthanes | 2,5 | 31,5 | 35 | 30 | 4 |
| ci-dessus | 3,0 | 38 | 42 | 18 | " |
| | 3,5 | 44 | 49 | 18 | " |
| $H_2O$ = 145 ppm | | | | | |
| HCl = 18 ppm | | | | | |

La vitesse spatiale est exprimée en litre par heure et par litre de tamis.

On constate que HCl est retenu comme l'eau sur le tamis moléculaire.

EXEMPLE 6

On utilise un dispositif comme celui de la figure 1 dans lequel la colonne 40, de diamètre 800 mm comprend 35 plateaux à clapets et fonctionne sous 9 bars absolus. La température en tête est 40°C, la température en pied 110°C. Le desséchant, du $CaCl_2$ de teneur supérieure à 95 % est disposé en lit fixe dans une capacité (50), de diamètre 2800 m/m, hauteur 5800 m/m.

Le condenseur est alimenté par le $CH_3Cl$ sec sortie sécheur.

Le courant (6) contient :

10 à 20 % $CH_3Cl$

50-100 ppm $H_2O$

100-500 ppm $Cl_2$
50-500 ppm HCl
80 à 90 % $CH_2Cl_2+CHCl_3+CCl_4$

Le courant (8) constitue le reflux de la colonne à distiller comprenant du $CH_3Cl$, de l'HCl, du $Cl_2$ dont la teneur en $H_2O$ est inférieure à 20 ppm.

Le courant (7) représente le $CH_3Cl$ extrait de la colonne dont la composition est identique au flux (8).

Le courant (9) constitue la partie de $CH_3Cl$ recyclé, de même composition que les flux (7) et (8).

## Revendications

1. Procédé de séchage sélectif d'un mélange contenant au moins un hydrocarbure de l'acide chlorhydrique et de l'eau, caractérisé en ce qu'on le met en contact avec un desséchant choisi parmi (i) les sulfates, les chlorures ou les perchlorates métalliques anhydres ou (ii) le pentoxyde de phosphore jusqu'à ce que le desséchant ait retenu seulement la majeure partie de l'eau.

2. Procédé elon la revendication 1, caractérisé en ce que l'hydrocarbure est un hydrocarbure chloré ayant un ou deux atomes de carbone.

3. Procédé selon la revendication 2, caractérisé en ce que l'hydrocarbure est un chlorométhane.

4. Procédé selon l'une des revendications 1 à 3, caractérisé en ce que le desséchant est du chlorure de calcium.

5. Procédé de synthèse de chlorométhanes, caractérisé en ce qu'on met en contact en un point quelconque du procédé, un mélange contenant au moins un chlorométhane de l'acide chlorhydrique et de l'eau avec un desséchant choisi parmi (i) les sulfates, les chlorures ou les perchlorates métalliques anhydres ou (ii) le pentoxyde de phosphore.

6. Procédé selon la revendication 5, caractérisé en ce que le chlorométhane est essentiellement du chlorure de méthyle.

7. Procédé selon la revendication 6, caractérisé en ce que le mélange constitué essentiellement de chlorure de méthyle d'acide chlorhydrique et d'eau est la phase gazeuse issue d'une colonne de séparation du chlorure de méthyle des chlorométhanes supérieurs.

8. Procédé selon l'une des revendications 5 à 7, caractérisé en ce que le desséchant est du chlorure de calcium.

## Patentansprüche

1. Verfahren zur selektiven Trocknung eines aus mindestens einem Kohlenwasserstoff bestehenden Gemisches von Chlorwasserstoffsäure und Wasser, dadurch gekennzeichnet, daß es mit einem unter (i) den wasserfreien Metallsulfaten, -chloriden oder -perchloraten oder (ii) Phosphorpentoxid ausgewählten Trockenmittel in Berührung gebracht wird, bis das Trockenmittel lediglich den größten Teil des Wassers aufgenommen hat.

2. Verfahren nach Anspruch 1, dadurch gekennzeichnet, daß der Kohlenwasserstoff ein chlorierter Kohlenwasserstoff mit ein oder zwei Kohlenstoffatomen ist.

3. Verfahren nach Anspruch 2, dadurch gekennzeichnet, daß der Kohlenwasserstoff ein chloriertes Methan ist.

4. Verfahren nach einem der Ansprüche 1 bis 3, dadurch gekennzeichnet, daß das Trockenmittel Calciumchlorid ist.

5. Verfahren zur Synthese von chlorierten Methanen, dadurch gekennzeichnet, daß an einer beliebigen Stelle des Verfahrens ein aus mindestens einem chlorierten Methan, Chlorwasserstoffsäure und Wasser bestehendes Gemisch mit einem unter (i) den wasserfreien Metallsulfaten, -chloriden oder - perchloraten oder (ii) Phosphorpentoxid ausgewählten Trockenmittel in Berührung gebracht wird.

6. Verfahren nach Anspruch 5, dadurch gekennzeichnet, daß das chlorierte Methan wesentlich Methylchlorid ist.

7. Verfahren nach Anspruch 6, dadurch gekennzeichnet, daß das wesentlich aus Methylchlorid, Chlorwasserstoffsäure und Wasser bestehende Gemisch die Gasphase ist, die aus einer Kolonne zur Abtrennung des Methylchlorides von den höher chlorierten Methanen austritt.

8. Verfahren nach einer der Ansprüche 5 bis 7, dadurch gekennzeichnet, daß das Trockenmittel Calciumchlorid ist.

## Claims

1. Process for the selective drying of a mixutre containing at least one hydrocarbon, hydrochloric acid and water, characterised in that it is brought into contact with a drying agent chosen from (i) the anhydrous metal sulphates, chlorides or perchlorates or (ii) phosphorus pentoxide until the drying agent has retained only the majority of the water.

2. Process according to Claim 1, characterised in that the hydrocarbon is a chlorinated hydrocarbon having one or two carbon atoms.

3. Process according to Claim 2, characterised in that the hydrocarbon is a chloromethane.

4. Process according to one of Claims 1 to 3, characterised in that the drying agent is calcium chloride.

5. Process for the synthesis of chloromethanes, characterised in that a mixture containing at least one chloromethane, hydrochloric acid and water is brought into contact, at any point of the process, with a drying agent chosen from (i) the anhydrous metal sulphates, chlorides or perchlorates or (ii) phosphorus pentoxide.

6. Process according to Claim 5, characterised in that the chloromethane is essentially methyl chloride.

7. Process according to Claim 6, characterised in that the mixture consisting essentially of methyl chloride, hydrochloric acid and water is the gaseous phase emerging from a column for separating methyl chloride from the higher chloromethanes.

8. Process according to one of Claims 5 to 7, characterised in that the drying agent is calcium chloride.

FIGURE 1